# EUROPÄISCHE PATENTSCHRIFT

(11) **EP 1 507 472 B1**
(45) Veröffentlichungstag und Bekanntmachung des Hinweises auf die Patenterteilung: **14.11.2007**
(21) Anmeldenummer: 03752717.3
(22) Anmeldetag: 29.04.2003
(51) Int. Cl.: A61B 1/00

(54) **ANORDNUNG ZUR INTRAOPERATIVEN FESTLEGUNG DER LAGE EINES GELENKERSATZIMPLANTATS**
ARRANGEMENT FOR INTEROPERATIVE DETERMINATION OF THE POSITION OF AN ARTICULAR JOINT REPLACEMENT IMPLANT
DISPOSITIF DE FIXATION INTRA-OPERATOIRE DE L'EMPLACEMENT D'UN IMPLANT D'ARTICULATION ARTIFICIELLE

(30) Priorität: 21.05.2002 DE 10222415; 18.02.2003 DE 10306793
(43) Veröffentlichungstag der Anmeldung: 23.02.2005
(73) Patentinhaber: Plus Orthopedics AG, 6343 Rotkreuz (CH)
(72) Erfinder: STIFTER, Jan, CH-5424 Unterehrendingen (CH); BROERS, Holger, 26670 Uplengen (DE)
(74) Vertreter: Popp, Eugen
(86) Internationale Anmeldenummer: PCT/EP2003/004469
(87) Internationale Veröffentlichungsnummer: WO 2003/096870

(56) Entgegenhaltungen:
- WO-A-02/17798
- WO-A-02/37935
- WO-A-99/23956
- US-A- 5 880 976
- WAHRBURG J ET AL: "Using robots to increase the accuracy of surgical interventions" INDUSTRIAL ELECTRONICS SOCIETY, 1998. IECON '98. PROCEEDINGS OF THE 24TH ANNUAL CONFERENCE OF THE IEEE AACHEN, GERMANY 31 AUG.-4 SEPT. 1998, NEW YORK, NY, USA,IEEE, US, 31. August 1998 (1998-08-31), Seiten 2512-2516, XP010308360 ISBN: 0-7803-4503-7

## Beschreibung

Die Erfindung betrifft eine Anordnung zur intraoperativen Festlegung der Lage eines Gelenkersatzimplantates, insbesondere einer Hüft- oder Schultergelenkpfanne oder eines zugehörigen Schaftimplantates oder eines Wirbelkörperersatzimplantats, unter Einsatz eines computertomographischen Verfahrens.

Chirurgische Eingriffe zur Ersetzung von Gelenken oder Gelenkbestandteilen beim Menschen sind seit langem bekannt und gehören in den Industrieländern zum klinischen Alltag. Es gibt auch seit Jahrzehnten eine intensive Entwicklungsarbeit zur Bereitstellung und fortlaufenden Verbesserung entsprechender Implantate, insbesondere von Hüftgelenksimplantaten, zunehmend aber auch von Knie-, Schulter- und Ellenbogengelenkimplantaten sowie auch von Wirbelkörperersatzimplantaten. Parallel zu diesen Entwicklungen, die inzwischen zu einer fast unübersehbaren Vielfalt von derartigen Implantatkonstruktionen geführt haben, werden auch geeignete Operationstechniken und -hilfsmittel bereitgestellt und fortentwickelt, insbesondere auch an die jeweiligen Implantatkonstruktionen angepasste Werkzeuge zum Einsetzen derselben.

Es versteht sich auch, dass Gelenkersatzoperationen die Gewinnung geeigneter Abbildungen des jeweiligen Gelenkbereiches vorangeht, auf deren Grundlage der Operateur ein geeignetes Implantat und seine Operationstechnik festlegt. Wurden hierzu früher zumeist Röntgenaufnahmen verwendet, sind in den letzten Jahren mehr und mehr Computertomogramme zum alltäglichen Handwerkszeug des Operateurs geworden. Dessen ungeachtet ist der dauerhafte Erfolg von Gelenkersatzimplantationen auch heute noch eng mit der Erfahrung des Operateurs korreliert, was zu einem beträchtlichen Teil auf die nicht zu unterschätzenden Schwierigkeiten der adäquaten intraoperativen Nutzung bildlicher Darstellungen zur optimalen Ausrichtung der Teile des Gelenkimplantates in Bezug auf die effektiven Rotationszentren und Belastungsachsen des einzelnen Patienten zurückzuführen ist.

Es hat daher in den letzten Jahren verstärkt Bemühungen zur Bereitstellung entsprechender Positionierungs-Hilfsmittel und -Verfahren für den Operateur gegeben, die im wesentlichen aus Entwicklungen auf dem Gebiet der Roboter- und Handhabungstechnik abgeleitet sind.

Die EP 0 553 266 B1 oder US 5,198,877 beschreiben ein Verfahren und ein Gerät zur kontaktlosen dreidimensionalen Gestaltdetektierung, das Anregungen zur Entwicklung medizinischer "Navigations"systeme und -verfahren gegeben hat; vgl. auch die ausführlichen Literaturhinweise in diesen Druckschriften.

Aus der US 5,871,018 und der US 5,682,886 sind Verfahren zur Ermittlung der Belastungsachse des Femurs bekannt. Gemäß diesen Verfahren werden in einem ersten Schritt die Koordinaten des Femurs beispielsweise durch eine Computertomographieaufnahme ermittelt und in einem Computer abgespeichert. Mit Hilfe der abgespeicherten Daten wird dann ein dreidimensionales Computermodell des Femurs erstellt, und anhand dieses Modells werden die optimalen Koordinaten für das Ansetzen einer Lehre an den Knochen sowie einer anschließend einzusetzenden Knieprothese berechnet. Grundlage hierfür ist die Berechnung der Belastungsachse des Femurs.

Nach einer derartigen Simulation wird der Femur des Patienten fixiert, und mit einer Registrierungseinrichtung werden einzelne Punkte an der Femuroberfläche angetastet, um die Orientierung des Femurs für die durchzuführende Operation festzustellen. Dieses Abtasten des Knochens erfordert, dass entweder große Teile längs des Femurs möglichst bis zum Hüftgelenk hin offengelegt sein müssen, um deren Oberfläche mit der Registrierungseinrichtung abtasten zu können, oder eine Art Nadel zum Durchstechen der Haut bis auf den Knochen als Abtastinstrument benutzt wird. Da jedoch jeder operative Eingriff für den Patienten ein Risiko darstellt und Nadelstiche Blutergüsse und ein zusätzliches Infektionsrisiko in den Knochenpartien verursachen, ist es nicht wünschenswert, einen zusätzlichen operativen Eingriff im Bereich der Hüfte vorzunehmen oder Nadeleinstiche entlang des Femurs durchzuführen, um den Ort des Rotationszentrums festzustellen. Des weiteren ist eine strenge Fixierung des Femurs auf einem Messtisch der Registrierungseinrichtung notwendig, da ansonsten Verschiebungen der Hüftpfanne während der Antastprozedur auftreten und die Schnittlehre nach erfolgter Registrierung der Femurkoordinaten u.U. falsch angesetzt werden würde.

Die FR 2 785 517 beschreibt ein Verfahren und eine Vorrichtung zum Detektieren des Rotationszentrums des Femurkopfes in der Hüftpfanne. Hierfür wird der Femur mit seinem Femurkopf in der Hüftpfanne bewegt, und die in verschiedenen Stellungen des Femurs aufgenommenen Messpunktkoordinaten werden abgespeichert. Sobald eine Verschiebung des Rotationszentrums des Femurs auftritt, wird ein entsprechender Gegendruck auf den Femurkopf ausgeübt, der bei der Bestimmung eines Punktes, der in Beziehung mit der Anordnung des Femurs steht, mit berücksichtigt wird.

In der DE 197 09 960 A1 werden ein Verfahren und eine Vorrichtung zur präoperativen Bestimmung der Positionsdaten von Endoprothesenteilen eines mittleren Gelenkes relativ zu den das mittlere Gelenk ausbildenden Knochen beschrieben. Hierbei wird vorgeschlagen, dass man durch Bewegung der Knochen um jeweils ein äußeres Gelenk, das sich an dem dem mittleren Gelenk abgewandten Ende des jeweiligen Knochens befindet, jeweils einen äußeren Gelenkpunkt bestimmt, dass man im Bereich des besagten mittleren Gelenkes für jeden der beiden Knochen ebenfalls einen Gelenkpunkt bestimmt, dass man durch geradlinige Verbindung der beiden so gefundenen Gelenkpunkte für jeden der beiden Knochen eine für diesen charakteristische Richtung bestimmt und schließlich die Orientierung der Endoprothesenteile relativ zu dieser charakteristischen Richtung bestimmt.

Ähnliche medizinische "Navigations"verfahren werden in der WO 95/00075 und der WO 99/23956 beschrieben, wobei Bilderfassungssysteme der oben erwähnten Art zur Positionserfassung von Referenzen an dem jeweiligen Gelenk benachbarten Knochen eingesetzt werden und aus der mit ihrer Hilfe gewonnenen virtuellen Repräsentation des Knochens bzw. Gelenkes charakteristische Punkte bzw. Achsen abgeleitet werden können.

Ein hinsichtlich der Zuverlässigkeit und insbesondere der Unabhängigkeit von intraoperativen Bewegungen des Patienten verbessertes und für den unmittelbaren Einsatz während der Operation vorgesehenes System dieser Art gemäß Oberbegriff des Anspruchs 1 ist Gegenstand der auf die Anmelderin zurückgehenden WO 02/17798 A1.

Der Erfindung liegt, ausgehend vom Stand der Technik, die Aufgabe der Bereitstellung einer für den Operateur schnell und einfach sowie mit sehr geringem Fehlerrisiko bedienbaren Anordnung dieser Art zugrunde, die die Erzielung deutlich verbesserter Operationsergebnisse ermöglicht.

Diese Aufgabe wird durch eine Anordnung mit den Merkmalen des Anspruchs 1 gelöst. Zweckmäßige Ausgestaltungen des Erfindungsgedankens sind Gegenstand der Unteransprüche. Deren Gegenstände liegen - in beliebiger Kombination miteinander - auch in modifizierten Ausführungen im Bereich der vorliegenden Erfindung.

Die Erfindung schließt den wesentlichen Gedanken der Bereitstellung einer integrierten Anordnung zur intraoperativen Festlegung der räumlichen Position und Winkellage eines Gelenkersatzimplantates ein, die im wesentlichen durch eine Computertomographie-Modellbildungseinrichtung, eine optische Koordinatenmessanordnung zur Bereitstellung von Realpositionskoordinaten von für die Operation relevanten Punkten bzw. Lagebeziehungsvektoren des engeren (oder auch weiteren) Gelenkbereiches und eine Matching-Verarbeitungseinheit zur Reälpositionsanpassung des CT-Abbildes einschließt. Zur Erfindung gehört weiter der Gedanke, dass die letztgenannte Komponente des Systems zur Berechnung von Transformationsparametern nach dem Prinzip der Minimierung der Normalenabstände ausgebildet ist.

In einer bevorzugten Ausführung ist die Matching-Verarbeitungseinheit zur Ausführung eines interaktiven Ausgleichungsverfahrens zur Anpassung einer angetasteten Knochenoberfläche an eine korrespondierende virtuelle Oberfläche des Abbildes unter kombinierter Anwendung des Prinzips der Dreiecksvermaschung und eines räumlichen Spline-Ansatzes mit der Bestimmtheit der Unbekannten als Spline-Parameter ausgebildet. Diese Ausführung vermeidet weitgehend die Nachteile, die einer reinen Dreiecksvermaschung einerseits und dem räumlichen Splineansatz andererseits anhaften, nämlich einerseits das Auftreten von Sprüngen und Kanten bei der Generierung einer Oberfläche eines 3-D-Modells und übermäßige Schwingungen in Randbereichen andererseits. Beim hier favorisierten kombinierten Verfahren wird speziell in Randbereichen und schlecht bestimmten Bereichen die Oberfläche mit den Verfahren der Dreiecksvermaschung generiert.

In einer auf den praktischen Operationseinsatz zugeschnitten Ausführung hat die Anordnung eine mit der Computertomografie-Modellbildungseinrichtung verbundene, insbesondere als interaktive Benutzerschnittstelle mit Mitteln zur Benutzerführung ausgebildete, Eingabeschnittstelle zur Eingabe von Implantatparametern einer vorbestimmten Menge von einsetzbaren Implantaten und zur Vorgabe möglicher Implantatpositionen und -ausrichtungen bezüglich des Abbildes. Hierbei ist die Matching-Verarbeitungseinheit mit der Eingabeschnittstelle verbunden und zur Bestimmung von Sollkoordinaten bzw. eines Soll-Bewegungsvektors des einzusetzenden Implantats sowie eines Resektionsbereiches bzw. Resektionsinstrumentes für dieses aus mindestens einem Satz eingegebener Implantationsparameter, -positionen und -ausrichtungen ausgebildet. Speziell ist die Eingabeschnittstelle zur Eingabe und Abbild-Integration der relevanten Körperachsenvektoren und der Implantatparameter einer Hüftgelenkpfanne, insbesondere der Koordinaten des Rotationszentrums sowie des Anteversionswinkels und des Abduktionswinkels, ausgebildet.

Mit dieser Ausführung wird dem Operateur also eine hochentwickelte Benutzerführung zur weitgehend automatisierten Festlegung einer bezüglich der individuellen anatomischen Verhältnisse optimalen Position und Ausrichtung des Gelenkersatzimplantates zur Verfügung gestellt. Er kann damit auf der Grundlage von vorab gewonnenen Computertomogrammen und einer auf dieser Grundlage erstellten OP-Planung die wesentlichen intraoperativ zu treffenden Entscheidungen computerbasiert ableiten, wodurch eine deutlich höhere Genauigkeit erreicht wird und eklatante Fehlpositionierungen praktisch völlig ausgeschlossen werden können. Zweckmäßigerweise umfasst die optische Koordinatenmessanordnung neben der Stereokamera oder -Kameraanordnung einen ersten Mehrpunktgeber, der als beweglicher, handgeführter Taster zum Antasten knöcherner Referenzen im Gelenk- bzw. Wirbelbereich zur Bestimmung von deren Koordinaten ausgebildet ist. Ein zweiter Mehrpunktgeber ist zur starren Befestigung an einem Knochen oder Wirbelkörper im Gelenk- bzw. Wirbelbereich ausgebildet.

Im Sinne der Bereitstellung einer integrierten Gesamtanordnung gehört hierzu auch ein Resektionsinstrument, insbesondere ein Fräser oder eine Raspel, das mit dem zweiten oder einem dritten Mehrpunktgeber starr zu einer geometrisch kalibrierten, navigierbaren Werkzeug-Geber-Einheit verbindbar ist. Aus den Gebersignalen dieser Einheit sind Realpositionskoordinaten eines Wirkabschnitts des Resektionsinstruments, insbesondere eines Fräserkopfes bzw. Raspelabschnittes, und hieraus wahlweise Realpositionskoordinaten eines mit dem Resektionsinstrument geschaffenen Resektionsabschnittes bestimmbar. Hierzu gehört dann eine Ausbildung der Eingabeschnittstelle zur Eingabe von Instrumentparametern des Resektionsinstrumentes, die dessen synoptische Darstellung mit dem durch die Computertomografie-Modellbildungseinrichtung gewonnenen Abbild des Gelenk- bzw. Wirbelbereiches erlauben. Weiterhin zeichnet sich diese Ausführung aus durch eine Ausbildung der Matching-Verarbeitungseinheit zur Zuordnung der Realpositionskoordinaten des Wirkabschnittes und wahlweise der Realpositionskoordinaten des Resektionsabschnittes zum Abbild des Gelenk- bzw. Wirbelbereiches im wesentlichen in Echtzeit. Schließlich umfasst diese Anordnung eine zur synoptischen Darstellung des Wirkabschnittes bzw. Resektionsabschnittes in seiner aktuellen Position mit dem an Realpositionskoordinaten angepassten Abbild des Gelenk- bzw. Wirbelbereiches ausgebildete Bilddarstellungseinheit.

In einer weiteren Fortbildung des Erfindungsgedankens gehört zur Gesamtanordnung weiterhin ein Montagewerkzeug, insbesondere Schraubwerkzeug, das mit dem zweiten oder dritten Mehrpunktgeber starr zu einer geometrisch kalibrierten, navigierbaren Werkzeug-Geber-Einheit verbindbar ist. Aus den Gebersignalen dieser Einheit sind Realpositionskoordinaten eines Wirkabschnitts des Montagewerkzeuges und damit wahlweise des Implantats selbst bestimmbar. Weiterhin zeichnet sich diese Anordnung aus durch eine Ausbildung der Eingabeschnittstelle zur Eingabe von Werkzeugparametern des Montagewerkzeugs, die dessen synoptische Darstellung mit dem durch die Computertomografie-Modellbildungseinrichtung gewonnenen Abbild des Gelenk- bzw. Wirbelbereiches erlauben, sowie eine Ausbildung der Matching-Verarbeitungseinheit zur Zuordnung der Realpositionskoordinaten des Wirkabschnittes und wahlweise des Implantats zum Abbild des Gelenk- bzw. Wirbelbereiches im wesentlichen in Echtzeit. Hier ist die Bilddarstellungseinheit dann zur synoptischen Darstellung des Wirkabschnittes bzw. Implantats in seiner Realposition mit dem an Realpositionskoordinaten angepassten Abbild des Gelenk- bzw. Wirbelbereiches ausgebildet.

Bei den vorgenannten Ausführungen ist das Resektionsinstrument und/oder das Montagewerkzeug als handgeführtes Werkzeug mit einer Handhabe ausgebildet, welche einen Befestigungsabschnitt zur starren Verbindung mit dem Mehrpunktgeber aufweist. Es versteht sich, dass bei Implantatsystemen, zu denen mehrere Resektions- oder Montagewerkzeuge gehören, diese in vorteilhafter Weise sämtlich einen entsprechenden Befestigungsabschnitt haben sollten, um eine über alle Resektions- bzw. Montageschritte durchgängige computerbasierte Navigierung bereitzustellen.

In einer weiteren sinnvollen Fortbildung des Erfindungsgedankens umfasst die Gesamtanordnung ein Adapterteil zur starren Befestigung eines Mehrpunktgebers an dem Gelenkersatzimplantat, insbesondere am proximalen Ende eines Schaftimplantats, zur Bildung einer navigierbaren Implantat-Geber-Einheit. Aus den Gebersignalen dieser Einheit sind Realpositionskoordinaten des Adapters und damit wahlweise des Implantats selbst bestimmbar. Diese Ausführung zeichnet sich aus durch eine Ausbildung der Eingabeschnittstelle zur Eingabe von Adapterparametern, die die synoptische Darstellung des Adapters bzw. des Implantats mit dem durch die Computertomografie-Modellbildungseinrichtung gewonnenen Abbild des Gelenk- bzw. Wirbelbereiches erlauben, und eine Ausbildung der Matching-Verarbeitungseinheit zur Zuordnung der Realpositionskoordinaten des Adapters und wahlweise des Implantats zum Abbild des Gelenk- bzw. Wirbelbereiches im wesentlichen in Echtzeit. Hier ist dann - analog zu den vorgenannten Ausführungen - die Bilddarstellungseinheit zur synoptischen Darstellung des Adapters bzw. Implantats in seiner Realposition mit dem an Realpositionskoordinaten angepassten Abbild des Gelenk- bzw. Wirbelbereiches ausgebildet.

Der oder die Mehrpunktgeber ist/sind vorzugsweise als passiver Vierpunktgeber mit vier Kungelreflektorabschnitten ausgebildet. Der Stereokamera bzw. Kameraanordnung ist hierbei eine Beleuchtungseinrichtung zugeordnet, mit der der oder die Mehrpunktgeber angeleuchtet werden, so dass definierte Reflexe zur "Abbildung" des jeweiligen Mehrpunktgebers zur Verfügung stehen. Zur weitgehenden Ausschaltung von den Operateur störendem Licht arbeitet die Beleuchtungseinrichtung bevorzugt im Infrarotbereich.

Zur Gewährleistung des Einsatzes aller relevanten Implantatkonstruktionen bei einer mit der vorgeschlagenen Anordnung ausgeführten Operation hat die Benutzerschnittstelle einen Mehrbereichsspeicher zur Speicherung der Implantatparameter der einsetzbaren Implantate oder eine Datenbank-Schnittstelle zu einer Implantatparameter-Datenbank. Weiterhin hat, wie oben bereits angesprochen, die Benutzerschnittstelle Mittel zur Realisierung einer Menüführung, und diese sind hier zur Ausführung eines interaktiven Auswahlvorganges einer Komponente unter mehrfachem Zugriff auf den Mehrbereichsspeicher bzw. die Implantatparameter-Datenbank ausgebildet.

Eine den Operateur besonders weitgehend unterstützende Ausführung der vorgeschlagenen Anordnung umfasst eine mit der Auswertungseinheit und der Matching-Verarbeitungseinheit verbundene Steuersignal-Erzeugungseinheit. Diese ist zu einem Vergleich eines Satzes von über die Eingabeschnittstelle eingegebenen und an die Realpositionskoordinaten des Gelenk- bzw. Wirbelbereiches angepassten Implantatpositionsdaten bzw. -ausrichtungsdaten mit aktuell erfassten Realpositionskoordinaten des Wirkabschnitts des Resektionsinstruments oder Montagewerkzeugs oder Implantats und zur Bestimmung einer Abweichung zwischen Soll- und Ist-Positionskoordinaten und zur Ausgabe von Abweichungsdaten oder eines aus der Abweichung abgeleiteten Steuerbefehls, insbesondere per Text- oder Sprachausgabe und/oder in einer synoptischen Darstellung mit dem Abbild, ausgebildet.

Nachfolgend wird - zunächst ohne Bezugnahme auf konkrete Anordnungsaspekte - eine sinnvolle Verfahrensdurchführung zur Vorbereitung einer CT-basierten Hüftgelenkimplantation zusammenhängend beschrieben.

Grundsätzlicher Ablauf:
1. CT-Scan des Patienten
2. Berechnung 3D-Modell aus CT-Daten
3. Planung in CT-Slices und im 3D-Modell
4. Messung (Festlegung) des Körperachsenkoordinatensystems
5. Transformation des Modells in das Körperachsenkoordinatensystem
6. Antasten der Oberfläche des Acetabulums relativ zum knochenfesten Locator
7. Berechnung der Transformationsparameter zwischen 3D-Planung und knochenfestem Adapter durch Minimierung der Normalenabstände
8. Export der Planungsdaten in die OP (Koordinatensystem der knochenfesten Locatoren)
9. Ausrichtung der kalibrierten Instrumente.

Um die Position der Pfanne dreidimensional planen zu können, wird von der Hüfte des Patienten ein CT aufgenommen. Die Knochenstruktur wird aus den einzelnen Schnittbildern extrahiert und ein 3D-Modell der Hüfte berechnet, in dem die Lage und Ausrichtung der künstlichen Pfanne geplant wird und die anatomischen Körperachsen gemessen werden.

Die Lage und Ausrichtung der Implantatsteile werden der weiterverarbeitenden Navigationssoftware als zu realisierende Implantatssolllage zur Verfügung gestellt. Die Planungsdaten beinhalten folgende Informationen:
- Lage der Körperachsen im 3D-Modell
- Geplantes Rotationszentrum des künstlichen Hüftgelenks
- Antetorsionswinkel und Abduktionswinkel der geplanten Pfanne

Während der Operation wird zunächst am Beckenkamm ein knochenfester Lokator als Beckenreferenzkoordinatensystem angebracht. Daraufhin wird ein Zugang geschaffen und der Femurkopf reseziert. Ziel des weiteren Vorgehens ist, das Modell, in dem die Planung bekannt ist, in der tatsächlichen OP-Situation am Patienten wiederzufinden. Zu diesem Zweck werden mit einem Handtaster Punkte an der Knochenoberfläche der Hüfte angetastet. Die Abtastung erfolgt im wesentlichen im Bereich des Acetabulums, da hier die Knochenoberfläche durch die Resektion des Femurkopfes relativ gut zugänglich ist. In kleinerem Umfang werden weitere Punkte am Beckenkamm auf der Haut angetastet.

Da beide Datensätze - intraoperativ angetastete Punkte und 3D-Modell - keine bekannten identischen Punktinformationen enthalten, können diese nicht direkt aufeinander transformiert werden. Die abgetastete Oberfläche wird deshalb in einem iterativen Matching-Verfahren an die Oberfläche des 3D-Modells angenähert. Die intraoperativ abgetastete Punktwolke wird näherungsweise über einen zu messenden Hilfsstrahl in das System der Körperachsen transformiert. Der Handtaster wird dabei näherungsweise in die Mitte des Aceztabulums und in die Richtung des geplanten Pfannenimplantates gehalten und seine Position und Ausrichtung gemessen.

Beim anschließenden Matching wird zu jedem Punkt der angetasteten Punktwolke der Normalenvektor von der 3D-Oberfläche berechnet. Grundsätzliches Prinzip der Ausgleichung ist die Minimierung der Normalenabstände aller angetasteten Punkte zu der 3D-Oberfläche mit den Unbekannten der räumlichen 3D-Transformation der beiden Koordinatensysteme, einem konstanten Offset und der Oberflächenneigung als Gewicht. Das Matching liefert als Ergebnis die Transformationsparameter vom CT-Koordinatensystem zum hüftfesten Koordinatensystem.

Beim Matching wird zu jedem Punkt ein Normalenvektor auf der Fläche des 3D-Modells als lokal begrenzte räumliche Oberfläche berechnet. Ein Problem ist die Generierung der Oberfläche für die Berechnung der Normale auf der Oberfläche durch den Einzelpunkt. Bei der herkömmlichen Dreiecksvermaschung sind Sprünge und Kanten nicht zu vermeiden. Dies hat zur Folge, dass bei einer geringen Verschiebung der Oberfläche extreme Änderungen der Normalenrichtung auftreten würden. Dieser Effekt kann durch den räumlichen Splineansatz geglättet werden. Leider kommt es hierbei aber in nicht ausreichend definierten Bereichen (z.B. Rand) zu überzogenen Schwingungen, die sich dann von der wahren Oberfläche weit entfernen. Daher wurde die Bestimmtheit der Unbekannten der Spline-Parameter eingeführt, wodurch schlecht definierte Bereiche für die Berechnung ausgeklammert werden können. An diesen Stellen wird dann die Oberfläche mit der Dreiecksvermaschung generiert. Der Spline-Ansatz versagt insbesondere bei glatten Oberflächen, wo aber die Dreiecksvermaschung zu guten Ergebnissen führt.

Zusätzlich wird bei dem Matching ein konstanter Offset mitberechnet. Bei den Messungen der Oberflächenpunkte wird i.d.R. ein Handtaster mit einer Antastkugel verwendet, so dass auch bei eigentlich streng gleichen Oberflächen die Antastfläche um den Radius der Antastkugel verschoben gemessen wird. Also sinnvoll hat sich die Einführung eines Gewichtes für den Normalenvektor gezeigt. Je nach Lage des Normalenvektors erhält er ein höheres Gewicht aufgrund der Qualität der Unbekannten der Spline-Facette und der tatsächlichen Neigung des Vektors zu der Oberfläche.

Durch das Matching ist die Transformation der Planungsdaten in das knochenfeste System möglich, womit die Ausrichtung der Instrumente entsprechend zu den Planungsdaten vorgenommen werden kann. Hierzu sind die Instrumente entsprechend der Größen und der Implantatwahl zu kalibrieren. Die Lage eines Instruments wird im hüftfesten Koordinatensystem gemessen und mit Hilfe der aus dem Matching resultierenden Transformationsparameter in das Koordinatensystem der Körperachsen transformiert. Durch die Kalibrierung der Instrumente kann die Abweichung zwischen der Ist-Position und der Planungsposition angezeigt werden, intraoperativ kann nun online die Ist-Position in der Planung angezeigt und die Planungsposition in der Navigation verändert werden.

Das Vorgehen bei der Navigation des Schaftes kann analog zur CT-basierten Pfannennavigation ablaufen.
1. Daten aus dem CT-Scan des Patienten
2. Berechnung 3D-Modell aus CT-Daten
3. Planung in CT-Slices und im 3D-Modell
4. Transformation des Modells in das Körperachsenkoordinatensystem
5. Export der Planungsdaten in die Navigation
6. Oberfläche des Femurs relativ zum knochenfesten Femurlocator intraoperativ antasten
7. Transformationsparameter durch Minimierung der Normalenabstände berechnen
8. Ausrichtung der kalibrierten Instrumente.

Vorteile und Zweckmäßigkeiten ergeben sich im übrigen aus der nachfolgenden Beschreibung eines bevorzugten Ausführungsbeispiels - einer Anordnung in Verbindung mit einem Verfahren zur Implantation eines künstlichen Hüftgelenkes - in Verbindung mit den Figuren. Von diesen zeigen:
- Fig. 1: einen Beckenkamm-Lokator mit zugehöriger Klammer (Adapter), aufgeklemmt auf einen Beckenkamm, in perspektivischer Darstellung,
- Fig. 2: zusätzlich einen Handtaster zum Antasten der Tischoberfläche zwecks Bestimmung der Tischebene sowie knöchener Referenzen am Beckenkamm (über der Haut), in perspektivischer Darstellung,
- Fig. 3: zusätzlich zum Beckenkamm-Lokator eine perspektivische Darstellung eines Femur-Lokators mit zugehöriger Klammer zur Fixierung im proximalen Bereich eines Femurs,
- Fig. 4: eine Kugeladapter-Handtaster-Kombination zur Bestimmung des Zentrums des Acetabulums in perspektivischer Darstellung,
- Fig. 5: eine perspektivische Darstellung einer Fräser-Lokator-Kombination zum Fräsen des Sitzes für eine Hüftpfanne,
- Fig. 6: eine schematische Ausschnittdarstellung eines Schirmbildes eines PC-Monitors zur visuellen Darstellung der Aussichten des Fräsers bezüglich des Beckens,
- Fig. 7: eine perspektivische Darstellung einer Setzinstrument-Lokator-Kombination zum Eindrehen einer künstlichen Hüftpfanne in den vorbereiteten Sitz und
- Fig. 8: eine perspektivische Darstellung einer Markkanalahlen-Lokator-Kombination zur Bestimmung des Verlaufes des Markkanals in einem Femur.

Die nachfolgende Darstellung wird primär anhand eines Vorgehens zur Bestimmung der relevanten geometrischen Größen sowie zur Implantation einer Hüftpfanne gegeben, ergänzend wird aber auch auf die - hiervon relativ unabhängige - Bestimmung der relevanten geometrischen Größen und die Ausführung der Implantation eines Schaftteiles als zweiter Komponente eines künstlichen Hüftgelenkes hingewiesen.

Der Operateur hat in der Planung einer Hüftgelenkimplantation für die Pfanne folgende Werte zu bestimmen:
1. Größe der künstlichen Pfanne
2. Inklinationswinkel und Antetorsionswinkel
   Die beiden Winkel der Ausrichtung der Pfannenachse relativ zu den Körperebenen werden hier vom Operateur nach medizinischen Gesichtspunkten im Röntgenbild gewählt. Diese Winkel können ebenfalls vom Operateur intraoperativ verändert werden.
3. Winkel in der sagittalen Körperebene zwischen vertikaler Achse und der Richtung vom Beckenkamm zur Symphyse.
   Durch die Bestimmung dieses Winkels wird eine intraoperative Bestimmung der Körperachsen und damit des Planungskoordinatensystems ermöglicht.

Es wird angenommen, dass der Patient sich zu Beginn der Operation in Rückenlage befindet; der Arzt hat ein Röntgenbild zur Verfügung, welches hinreichend die anatomische Gesamtkonstellation sowie die Knochenbeschaffenheit erkennen lässt. Er gewinnt daraus erste Vorstellungen hinsichtlich der einzusetzenden Implantatgröße und der zu bevorzugenden Grobausrichtung des Implantates. Es wird eine Inzision 3 - 5 cm dorsal der Spina Iliaca Superior Anterior mit einer Länge von 4 cm vorgenommen, der Beckenkamm freigelegt und das Gewebe mit dem Raspatorium freigelegt.

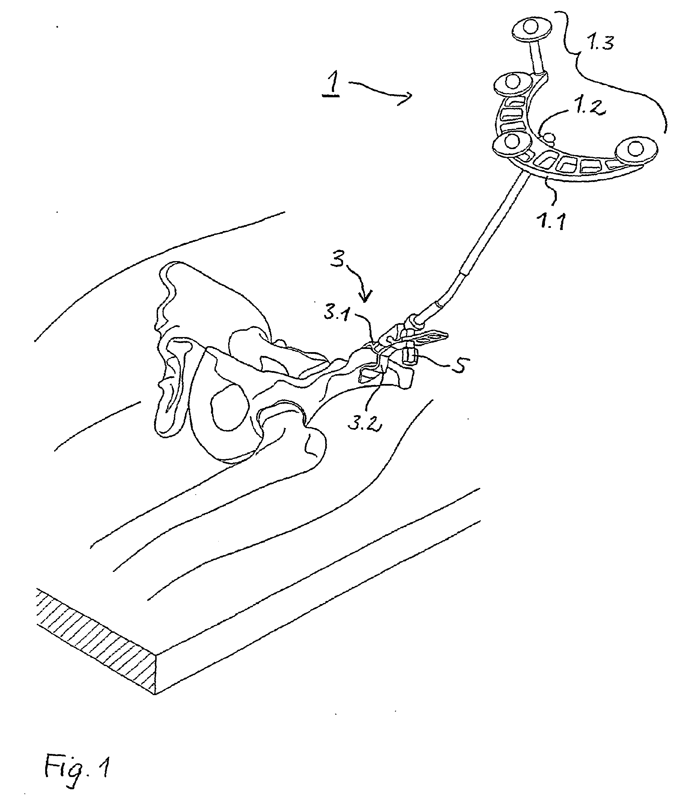

Fig. 1 zeigt einen Beckenkamm-Lokator 1 mit zugehöriger Befestigungsklammer 3, der im freigelegten Bereich des Beckenkammes angebracht wird. Die Befestigungsklammer 3 umfasst ein mediales Klammerteil 3.1 und ein laterales Klammerteil 3.2, die über eine Inbusschraube 5 miteinander verschraubt werden, bis die Befestigungsklammer fest am Beckenkamm sitzt. Der eigentliche Beckenkamm-Lokator 1 hat einen sichelförmig geschwungenen Grundkörper 1.1 mit einer Steckhülse 1.2 zum Aufstecken auf die Befestigungsklammer 3 sowie einem 4-Punkt-Lokatorfeld 1.3 aus vier IR-reflektierenden Kugeln, die jeweils partiell von einem (nicht gesondert bezeichneten) kugelabschnittförmigen Diffusor zur Vermeidung von Störstrahlungseinflüssen umgeben sind. Es handelt sich hierbei um sogenannte passive Targets oder Adapter, die als solche bekannt sind und deren Wirkungsweise in Verbindung mit einer - ebenfalls bekannten - Stereokameraanordnung eines sogenannten Navigationssystems daher hier nicht weiter beschrieben wird. Nach dem Aufstecken wird der Lokator 1 gegenüber der Befestigungsklammer 3 derart gedreht, dass das Lokatorfeld zur Kamera geeignet ausgerichtet ist, ohne dass eine der reflektierenden Kugeln durch eine andere abgeschirmt ist. Anschließend wird durch Verschrauben des Lokators mit der Befestigungsklammer eine starre Verbindung zwischen beiden hergestellt.

Statt am Beckenkamm kann der oben als Beckenkamm-Lokator bezeichnete Mehrpunktgeber 1 auch am Pfannendach des Beckens befestigt werden. Dies hat den Vorteil, dass die oben erwähnte (zusätzliche) Inzision im Bereich des Beckenkammes überflüssig wird, jedoch ist die Befestigung des Mehrpunktgebers - der dann als "OP-Feld-Lokator" bezeichnet wird - bei geschwächter Knochenstruktur weniger stabil.

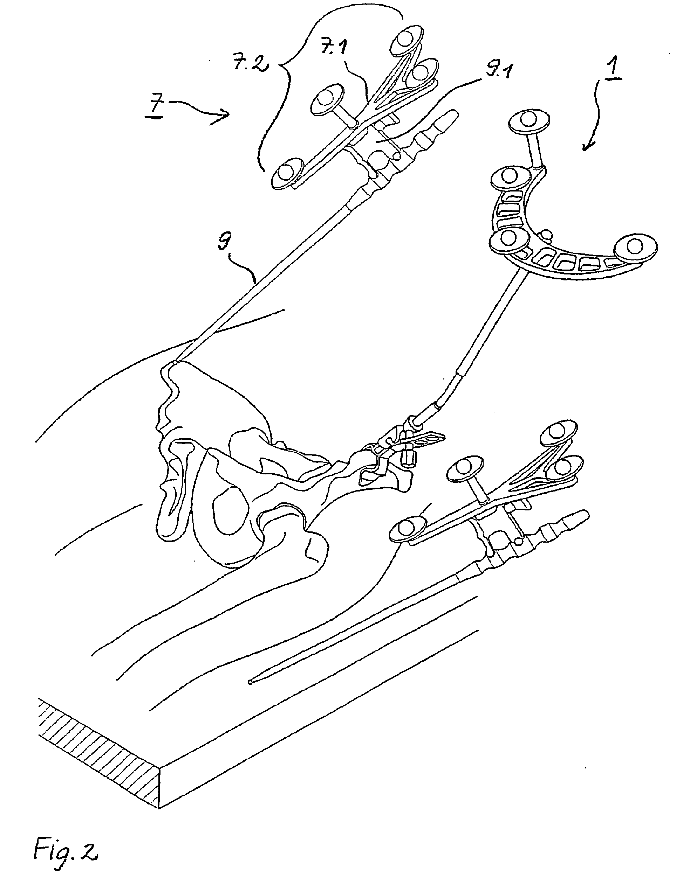

Fig. 2 zeigt neben dem oben beschriebenen skelettfesten Lokator 1 einen Handtaster 7 mit einem stabförmigen, sich zu einem Ende hin verjüngenden Tasterteil 9, von dem ein Halter 9.1 senkrecht absteht, einem annähernd Y-förmigen Taster-Grundkörper 7.1 und einem 4-Punkt-Lokatorfeld 7.2, ähnlich dem Aufbau des oben beschriebenen Beckenkamm-Lokators. Ähnlich sind auch die Lokatoren der weiter unten beschriebenen Anordnungskomponenten aufgebaut, so dass für diese die entsprechende Benennung der Teile bzw. Abschnitte und Beschreibung derselben fortgelassen wird.

Mit dem Handtaster 7 werden zu Beginn des Navigations-Ablaufes verschiedene Punkte der Ebene des OP-Tisches, auf dem der Patient liegt, angetastet, um eine Bestimmung der Lage der Tischebene im Raum zu ermöglichen. Diese wird zwar für die eigentliche Bestimmung der Patientenlagen nicht benötigt, kann aber für Plausibilitätsbetrachtungen (beispielsweise hinsichtlich der Bedeutung einer Beckenneigung des Patienten gegenüber der Tischebene etc.) herangezogen werden. Für die eigentliche Navigation wird üblicherweise angenommen, dass die Patienten-Frontalebene parallel zur Tischebene liegt.

Anschließend werden mit dem Handtaster 7 über der Haut charakteristische knöcherne Referenzen im Beckenbereich angetastet. Zunächst werden der linke und der rechte Beckenkamm sowie die Mitte der Symphyse angetastet. Durch diese drei angetasteten Punkte und den in der Planung ermittelten Kamm-Symphysen-Winkel können die Körperachsen eindeutig festgelegt werden. Die Richtung vom linken zum rechten Beckenkamm stellt die transversale Körperachse dar. Die Richtung von der Mitte der Beckenkammpunkte zur Symphyse wird um den Kamm-Symphysen-Winkel um die transversale Achse gedreht und stellt damit die vertikale Körperachse dar (orthogonal zur transversalen Achse). Die sagittale Körperachse ergibt sich aus den beiden erstgenannten Achsen als Orthogonale.

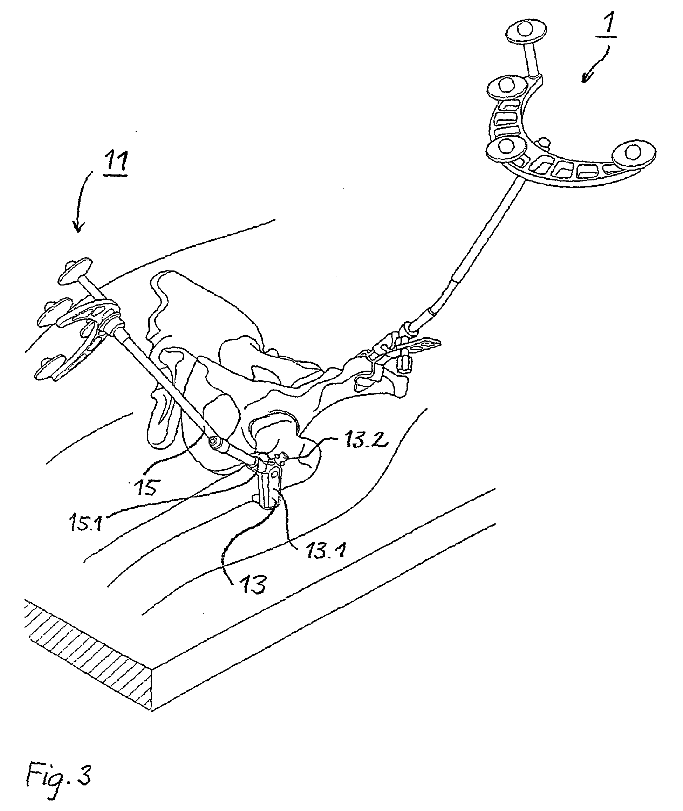

Fig. 3 zeigt zusätzlich zum Beckenkamm-Lokator 1 einen Femur-Lokator 11 mit zugehörigem Adapter (Femurklammer) 13 zur Befestigung nahe dem proximalen Ende des Femurs. Die Femurklammer 13 hat einen zweiteiligen Grundkörper aus einem in der Draufsicht gabelförmigen und in der Seitenansicht annähernd L-förmigen ersten Grundteil 13.1, von dem zwei Zapfen 13.2 zum Aufstecken des Lokators abstehen, und einem mit dem ersten Grundteil 13.1 verrastbaren, in der Seitenansicht annähernd L-förmiges zweiten Grundteil. Der Aufbau des Femur-Lokators 11 selbst entspricht - abgesehen von einem abgewinkelten Lokatorstab - im wesentlichen dem des Beckenkamm-Lokators.

Er wird über eine Steckhülse 15.1 am freien Ende eines Lokatorstabes 15 auf einen der beiden Zapfen 13.2 der Femurklammer 13 aufgesteckt.

Dann wird die Femurklammer 13 mit dem angesetzten Lokatorstab 15 auf der lateralen Femurseite etwa auf Höhe Trochanter Minor oder zwischen Trochanter Minor und Trochanter Major befestigt, indem die dort liegenden Muskelgruppen beiseite geschoben werden und die Klammer eingeschoben wird. Die Drehstellung ist so zu wählen, dass der Lokatorstab lateral aus dem OP-Feld, möglichst in Richtung der Kamera, ragt. Dann wird die Klammer mit mittlerem Drehmoment festgezogen, dass eigentliche (hier nicht gesondert bezeichnete) Lokatorfeld aufgesteckt und nach der Kamera ausgerichtet und schließlich der Femur-Lokator festgeschraubt.

Danach wird das kinematische Rotationszentrum der Hüfte sowohl im hüftfesten als auch im femurfesten Koordinatensystem durch mehrere relative Messungen des Femur-Lokators im hüftfesten Koordinatensystem bei unterschiedlichen Beinstellungen bestimmt. Die Transformation aller Messwerte kann somit vom hüftfesten Koordinatensystem in das Koordinatensystem der Körperachsen erfolgen. Hiermit können dann alle kalibrierten Werkzeuge zum Körperachsenkoordinatensystem ausgerichtet werden; siehe dazu weiter unten. Mit dem Rotationszentrum als Ursprung kann das Implantat an seinem kinematischen Ursprung eingesetzt werden. Sollten Korrekturen erforderlich sein, können Verschiebungen und Winkelveränderungen der Planung intraoperativ ausgeführt werden.

Nachdem der Operateur die Positionserfassungen in den verschiedenen Beinstellungen im "Dialog" mit der interaktiven Benutzerführung vorgenommen hat (wobei wieder eine Fehlerkorrektur aufgrund von Plausibilitätsberechnungen vorgesehen ist), wird der Femur-Lokator von der Klammer 13 abgenommen und der Femurkopf reseziert. Der Durchmesser des resezierten Kopfes wird gemessen und auf Grundlage des Messergebnisses eine geeignete Halbkugel für den nächsten Schritt, nämlich die Bestimmung des Zentrums des Acetabulums bzw. geometrischen Rotationszentrums der Hüfte, ausgewählt.

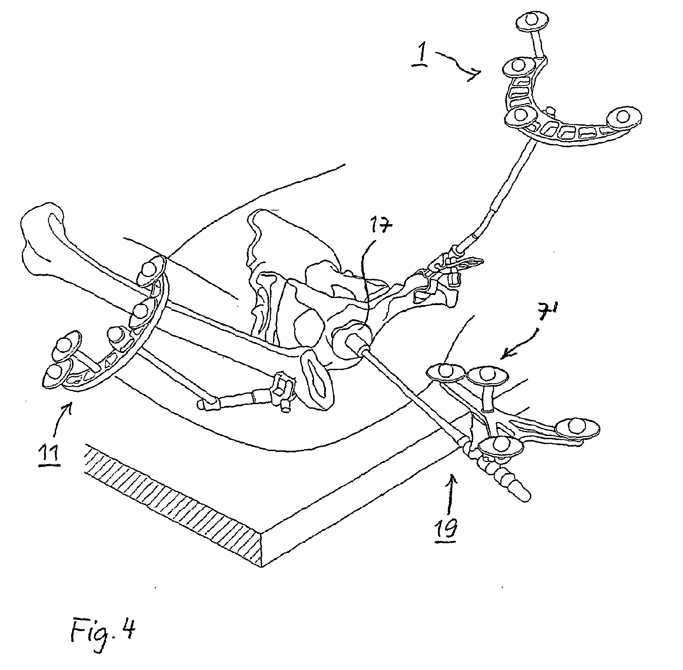

Wie Fig. 4 zeigt, wird die ausgewählte Halbkugel 17 mit einem Handtaster 7' der in Fig. 2 gezeigten und weiter oben beschriebenen Art zu einer Kugeladapter-Handtaster-Kombination 19 zusammengestellt. Durch Einsteuern dieses Lokators in den Pfannenbereich wird (üblicherweise unter Voraussetzung eines bestimmten Anteversionswinkels, z.B. 12°) zum einen die Gültigkeit des mittels des Femur-Lokators bestimmten (kinematischen) Rotationszentrums aus geometrischer Sicht überprüft, und zum anderen erlauben die Ergebnisse eine "Gegenprobe" zu der Implantations-Planungswerte unter geometrischen Gesichtspunkten. Außerdem lassen sich durch Bewegen der Halbkugel 17 im Pfannenbereich Hinweise auf mögliche mechanische Kollisionen gewinnen. Die Konstruktion der Halbschale und deren Adaption zum Handtaster realisiert die Antastspitze immer im Kugel-Zentrum der Antasthalbkugel.

Anschließend erfolgt im Rahmen des gespeicherten Auswertungsprogramms mit interaktiver Benutzerführung die endgültige Planung der Implantation, von der Bestimmung der einzusetzenden Implantatgröße bis hin zu Verschiebungswerten und Winkelgrößen. Das System errechnet auf dieser Grundlage sowie anhand von vorab eingegebenen spezifischen Instrumentdaten Sollpositionen für die einzusetzenden Resektions- bzw. Setzinstrumente oder, genauer gesagt, für deren Wirkabschnitte.

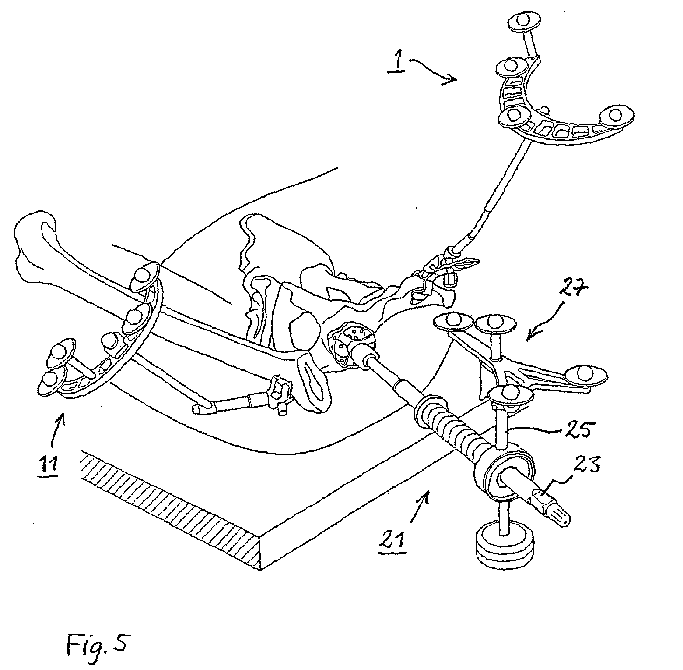

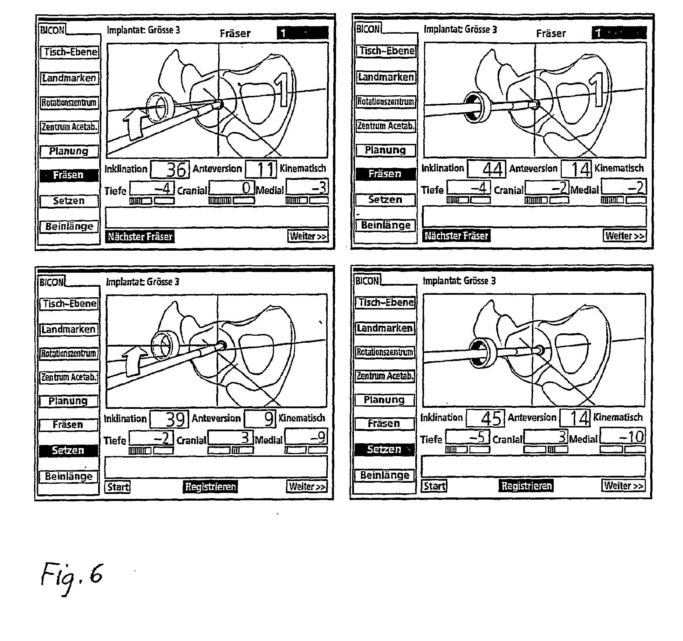

Fig. 5 zeigt neben Beckenkamm- und Femur-Lokator 1, 11 eine Fräser-Lokator-Kombination 21 mit einer Fräswelle 23, einem Fräswellenadapter 25 und einem Lokator 27, dessen Aufbau im wesentlichen denjenigen des Femur-Lokators 11 nach Fig. 3 entspricht. Dieses Instrument wird in der in der Figur ebenfalls gezeigten Weise in einem Pfannenbereich ausgerichtet, wobei die Lage und Ausrichtung aufgrund der Positionssignale aus dem Lokatorfeld erfasst und auf Schirmbildern in der in Fig. 6 gezeigten Weise visuell verdeutlicht wird. Eine entsprechend den Planungsdaten korrekte Lage des Fräsers wird durch einen um die Fräswelle sich erstreckenden Ring in der Displaydarstellung sowie durch akustische Signale angezeigt.

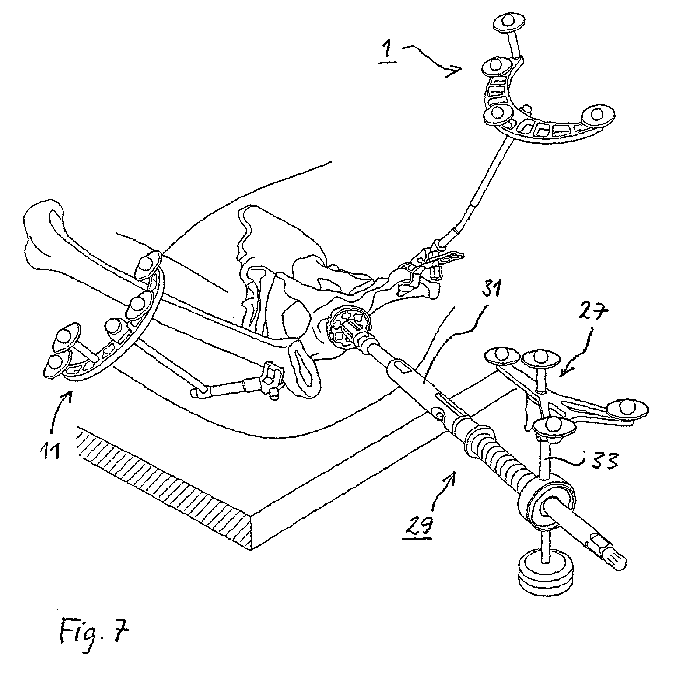

Sobald ein Pfannensitz gemäß den Planungsdaten hergestellt ist, wird die Fräser-Lokator-Kombination in eine Setzinstrument-Lokator-Kombination 29 umgebaut, wie sie in Fig 7 gezeigt ist. Hierbei wird wiederum der Lokator 27 eingesetzt, und zwar jetzt in Verbindung mit einem Setzinstrumentenschaft 31 und einem Schaftadapter 33. Mit diesem Instrument wird in einer zur Handhabung der Fräser-Lokator-Kombination weitgehend analogen und ebenso auf dem PC-Schirmbild dargestellten Art und Weise eine Hüftpfanne 35 gesetzt. Deren endgültige Position wird durch den Operateur noch in das System eingegeben.

Anschließend erfolgen die Schaftpräparation und -implantation (zunächst eines Testschaftes), und zwar entweder auf konventionelle oder ebenfalls durch das Navigationssystem gestützte Weise. Höhe und Anteversion des Schaftes sind dabei anhand der Planungsdaten fest eingestellt; lediglich die Kugelhalslänge ist noch frei wählbar. Dann wird das Gelenk mit dem Testschaft zusammengebaut und Stabilität sowie etwaige Kollisionen beim Bewegen des Schaftes in der Pfanne geprüft. Zudem erfolgt eine Grob-Prüfung der Beinlänge durch Vergleich der Lage der Knöchel des operierten des gesunden Beines. Falls Probleme bei der Gelenkstabilität auftreten, wird versucht, diese durch Wahl einer bestimmten Kugel oder eines Schaftes anderer Größe aus einem bereitstehenden Sortiment zu lösen.

Optional können in dieser Phase auch Messungen am anderen Bein unter Einsatz des Navigationssystem erfolgen, deren Ergebnisse im Sinne von Symmetriebetrachtungen zu einer Feinjustierung des Implantats herangezogen werden können. Er versteht sich, dass bei derartigen Messungen anstelle des oben beschriebenen Femur-Lokators ein zur äußeren Anbringung über der Haut modifizierter Femur-Lokator eingesetzt wird.

Ein wesentlicher Vorteil des vorgeschlagenen Systems besteht darin, dass unter Nutzung von Navigationsdaten auch ein Vorher-Nacher-Vergleich der Beinlängen (an der kranken Hüfte vor der Operation und während des oben erwähnten Überprüfungsschrittes in der Schlussphase der Operation) möglich ist. Hierzu wird der Femur-Lokator erneut auf dem am Femur verbliebenen Halter positioniert und fixiert und die Position bei gestrecktem, parallel zur Körperlängsachse ausgerichtetem Bein erfasst. Die gewonnenen Positionsdaten geben Hinweise auf eine etwaige Beinverlängerung bzw. Beinverkürzung sowie auch auf die sogenannte Lateralisierung bzw. Medialisierung, d.h. die "seitliche" Position des Femurs. Bei Hinweisen auf eine zu starke Medialisierung (Nach-Innen-Verschiebung) kann gegebenenfalls gegenüber dem Testschaft ein anderer Schaft in Verbindung mit einer anderen Kugel eingesetzt werden; in jedem Falle geben die Messwerte dem Arzt aber Hinweise, die bei der weiteren Versorgung des Patienten zu berücksichtigen sind.

Die nachfolgenden Anmerkungen beziehen sich auf eine Nutzung des beschriebenen Systems bei der Schaftpräparation und Implantation.

Für die Platzierung des Schaftes einer Hüftprothese ist die Herstellung eines geplanten Antetorsionswinkel des Femurhalses und die Winkelherstellung der ursprünglichen Beinlänge gefordert. Die Achsen-Ausrichtung des Schaftes richtet sich weitestgehend nach der Lage des Markkanals im Femur. Dies hat zur Folge, dass erst hieraus die eigentliche Schaftgröße bzw. dessen Offsets berechnet werden können.

Mit Hilfe einer kalibrierten Ahle wird der Markkanal des Femurs bestimmt. Eine weitere wichtige Information für die Platzierung des Schaftes ist die Bestimmung des Rotationszentrums; siehe dazu weiter oben.

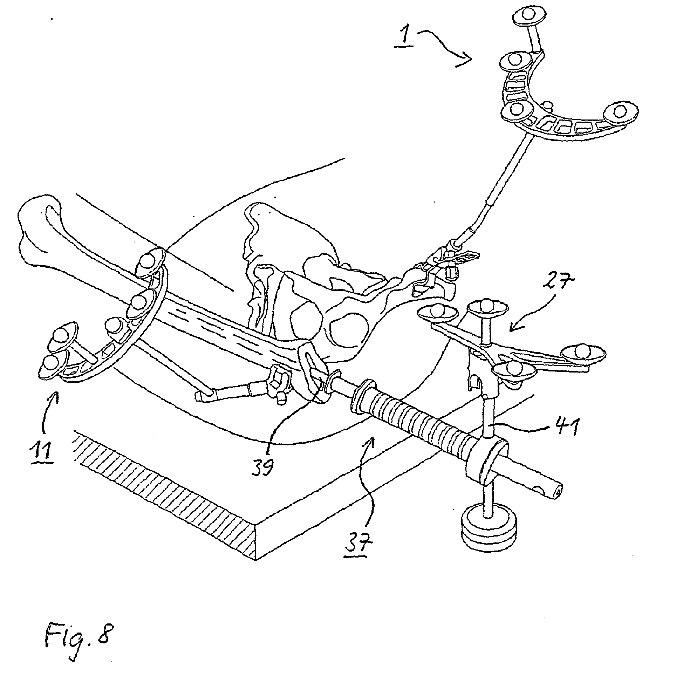

Fig. 8 zeigt eine hierzu einsetzbare weitere Komponente der vorgeschlagenen Anordnung, nämlich eine Markkanalahlen-Lokator-Kombination 37 mit einer Markkanalahle 39, einem Ahlenadapter 41 und (wiederum) einem Lokator 27, ähnlich der bereits in Fig. 3 gezeigten Lokatorausführung. Zum Einsetzen dieses Navigationsinstrumentes wird das proximale Femurende mit einem Kastenmeißel oder einer Stichsäge in der Nähe des Trochanter Major eröffnet und die Markkanalahle 39 dort von proximal eingeführt.

Präoperativ werden Inklinationswinkel und Antetorsionswinkel des Femurkopfes im Röntgenbild bestimmt und intraoperativ eingegeben. Zusätzlich ist die intraoperative Bestimmung des Antetorsionswinkel durch Messung von Landmarks am Kniegelenk und an dem oberen Sprunggelenk möglich, womit intraoperativ die Körperebenen bekannt sind. Die tatsächlichen Implantationswinkel und -positionen der Pfannennavigation können bei der Schaftimplantation mitberücksichtigt werden. Die letzte räumliche Position der Pfanne kann als relative Korrektur des Schaftes angebracht werden. Durch dieses Vorgehen ist eine optimale Implantation sichergestellt.

Die Präparation des Femurs zum Einsetzen des Schaftes erfolgt dann - analog zur Präparation des Pfannensitzes mit einem navigierten Fräser - mit einer navigierten Schaftraspel, d.h. einer Schaftraspel-Lokator-Kombination, welche der in Fig. 8 gezeigten Kombination sehr ähnlich ist und daher hier weder gezeigt noch genauer beschrieben wird. Nach der Präparation wird wiederum ein Testschaft eingesetzt und es werden die oben im Zusammenhang mit der pfannenseitigen Navigation beschriebenen Überprüfungen ausgeführt. Bei zufriedenstellenden Ergebnissen wird der endgültige Schaft dann eingesetzt, ohne dass dieser nochmals navigiert werden müßte.

### Bezugszeichenliste

- 1: Beckenkamm-Lokator
- 1.1: Grundkörper
- 1.2: Steckhülse
- 1.3: 4-Punkt-Lokatorfeld
- 3: Befestigungsklammer
- 3.1: Mediales Klammerteil
- 3.2: Laterales Klammerteil
- 5: Inbusschraube
- 7; 7': Handtaster
- 7.1: Taster-Grundkörper
- 7.2: 4-Punkt-Lokatorfeld
- 9: Tasterteil
- 9.1: Halter
- 11: Femur-Lokator
- 13: Femurklammer
- 13.1: erstes Grundteil
- 13.2: Zapfen
- 13.3: zweites Grundteil
- 15: Lokatorstab
- 15.1: Steckhülse
- 17: Halbkugel
- 19: Kugeladapter-Handtaster-Kombination
- 21: Fräser-Lokator-Kombination
- 23: Fräswelle
- 25: Fräswellenadapter
- 27: Lokator
- 29: Setzinstrument-Lokator-Kombination
- 31: Setzinstrumentenschaft
- 33: Schaftadapter
- 35: Hüftpfanne
- 37: Markkanalahlen-Lokator-Kombination
- 39: Markkanalahle
- 41: Ahlenadapter

## Patentansprüche

1. Anordnung zur intraoperativen Festlegung der räumlichen Position und Winkellage eines Gelenkersatzimplantats, insbesondere einer Hüft- oder Schultergelenkpfanne oder eines zugehörigen Schaftimplantats, oder eines Wirbelkörperersatzimplantats, insbesondere Lenden- oder Nackenwirbelimplantats, unter Einsatz eines computertomografischen Verfahrens, welche aufweist:
- eine Computertomografie-Modellbildungseinrichtung zur Erzeugung und Speicherung eines dreidimensionalen Abbildes eines mit dem Gelenkersatzimplantat zu versorgenden Gelenk- bzw. Wirbelbereiches,
- eine optische Koordinatenmessanordnung zur Bereitstellung von Realpositionskoordinaten definierter realer oder virtueller Punkte des Gelenk- bzw. Wirbelbereiches und/oder von Lagebeziehungsvektoren zwischen solchen Punkten innerhalb des Gelenk- bzw. Wirbelbereiches oder von diesen zu gelenkfunktionsrelevanten Punkten an einer Extremität außerhalb des Gelenk- bzw. Wirbelbereiches,
wobei die Koordinatenmessanordnung eine Stereokamera oder Stereokameraanordnung zur räumlichen Erfassung von Gebersignalen, mindestens einen Mehrpunktgeber, der eine Gruppe von miteinander starr verbundenen Messpunkten umfasst, und eine Auswertungseinheit zur Auswertung von durch den oder die Mehrpunktgeber gelieferten und durch die Stereokamera erfassten Messpunktkoordinatenmengen umfasst, und **gekennzeichnet durch**
- eine Matching-Verarbeitungseinheit zur Realpositionsanpassung des Abbildes an die tatsächliche aktuelle räumliche Lage des Gelenk- bzw. Wirbelbereiches anhand der Realpositionskoordinaten der definierten Punkte, wobei die Matching-Verarbeitungseinheit zur Berechnung von Transformationsparametern unter Minimierung der Normalenabstände ausgebildet ist.

2. Anordnung nach Anspruch 1,
**dadurch gekennzeichnet, dass**
die Matching-Verarbeitungseinheit zur Ausführung eines interaktiven Ausgleichungsverfahrens zur Anpassung einer angetasteten Knochenoberfläche an eine korrespondierende virtuelle Oberfläche des Abbildes unter kombinierter Anwendung des Prinzips der Dreiecksvermaschung und eines räumlichen Spline-Ansatzes mit der Bestimmtheit der Unbekannten als Spline-Parameter ausgebildet ist.

3. Anordnung nach Anspruch 1 oder 2,
**gekennzeichnet durch**
eine mit der Computertomografie-Modellbildungseinrichtung verbundene, insbesondere als interaktive Benutzerschnittstelle mit Mitteln zur Benutzerführung ausgebildete, Eingabeschnittstelle zur Eingabe von Implantatparametern einer vorbestimmten Menge von einsetzbaren Implantaten und zur Vorgabe möglicher Implantatpositionen und -ausrichtungen bezüglich des Abbildes,
wobei die Matching-Verarbeitungseinheit mit der Eingabeschnittstelle verbunden und zur Bestimmung von Sollkoordinaten bzw. eines Soll-Bewegungsvektors des einzusetzenden Implantats sowie eines Resektionsbereiches bzw. Resektionsinstrumentes für dieses aus mindestens einem Satz eingegebener Implantationsparameter, -positionen und -ausrichtungen ausgebildet ist.

4. Anordnung nach Anspruch 3,
**dadurch gekennzeichnet, dass**
die Eingabeschnittstelle zur Eingabe und Abbild-Integration der relevanten Körperachsenvektoren und der Implantatparameter einer Hüftgelenkpfanne, insbesondere der Koordinaten des Rotationszentrums sowie des Anteversionswinkeis und des Abduktionswinkels, ausgebildet ist.

5. Anordnung nach einem der vorangehenden Ansprüche,
**dadurch gekennzeichnet, dass**
ein erster Mehrpunktgeber der Koordinatenmessanordnung als beweglicher, handgeführter Taster zum Antasten knöcherner Referenzen im Gelenk- bzw. Wirbelbereich zur Bestimmung von deren Koordinaten ausgebildet ist.

6. Anordnung nach einem der vorangehenden Ansprüche,
**dadurch gekennzeichnet, dass**
ein zweiter Mehrpunktgeber zur starren Befestigung an einem Knochen oder Wirbelkörper im Gelenk- bzw. Wirbelbereich ausgebildet ist.

7. Anordnung nach einem der vorangehenden Ansprüche,
**gekennzeichnet durch**
- ein Resektionsinstrument, insbesondere einen Fräser oder eine Raspel, das mit dem zweiten oder einem dritten Mehrpunktgeber starr zu einer geometrisch kalibrierten, navigierbaren Werkzeug-Geber-Einheit verbindbar ist, derart, dass aus den Gebersignalen dieser Einheit Realpositionskoordinaten eines Wirkabschnitts des Resektionsinstruments, insbesondere eines Fräserkopfes bzw. Raspelabschnittes, und hieraus wahlweise Realpositionskoordinaten eines mit dem Resektionsinstrument geschaffenen Resektionsabschnittes bestimmbar sind,
- eine Ausbildung der Eingabeschnittstelle zur Eingabe von Instrumentparametern des Resektionsinstrumentes, die dessen synoptische Darstellung mit dem **durch** die Computertomografie-Modellbildungseinrichtung gewonnenen Abbild des Gelenk- bzw. Wirbelbereiches erlauben,
- eine Ausbildung der Matching-Verarbeitungseinheit zur Zuordnung der Realpositionskoordinaten des Wirkabschnittes und wahlweise der Realpositionskoordinaten des Resektionsabschnittes zum Abbild des Gelenk- bzw. Wirbelbereiches im wesentlichen in Echtzeit und
- eine zur synoptischen Darstellung des Wirkabschnittes bzw. Resektionsabschnittes in seiner aktuellen Position mit dem an Realpositionskoordinaten angepassten Abbild des Gelenk- bzw. Wirbelbereiches ausgebildete Bilddarstellungseinheit.

8. Anordnung nach einem der vorangehenden Ansprüche,
**gekennzeichnet durch**
- ein Montagewerkzeug, insbesondere Schraubwerkzeug, das mit dem zweiten oder dritten Mehrpunktgeber starr zu einer geometrisch kalibrierten, navigierbaren Werkzeug-Geber-Einheit verbindbar ist, derart, dass aus den Gebersignalen dieser Einheit Realpositionskoordinaten eines Wirkabschnitts des Montagewerkzeuges, insbesondere einer Schraubendreherklinge, und damit wahlweise des Implantats selbst bestimmbar sind,
- eine Ausbildung der Eingabeschnittstelle zur Eingabe von Werkzeugparametern des Montagewerkzeugs, die dessen synoptische Darstellung mit dem **durch** die Computertomografie-Modellbildungseinrichtung gewonnenen Abbild des Gelenk- bzw. Wirbelbereiches erlauben,
- eine Ausbildung der Matching-Verarbeitungseinheit zur Zuordnung der Realpositionskoordinaten des Wirkabschnittes und wahlweise des Implantats zum Abbild des Gelenk- bzw. Wirbelbereiches im wesentlichen in Echtzeit und
- eine zur synoptischen Darstellung des Wirkabschnittes bzw. Implantats in seiner Realposition mit dem an Realpositionskoordinaten angepassten Abbild des Gelenk- bzw. Wirbelbereiches ausgebildete Bilddarstellungseinheit.

9. Anordnung nach Anspruch 7 oder 8,
**dadurch gekennzeichnet, dass**
das Resektionsinstrument und/oder das Montagewerkzeug als handgeführtes Werkzeug mit einer Handhabe ausgebildet ist, welche einen Befestigungsabschnitt zur starren Verbindung mit dem Mehrpunktgeber aufweist.

10. Anordnung nach einem der Ansprüche 1 bis 7,
**gekennzeichnet durch**
- ein Adapterteil zur starren Befestigung eines Mehrpunktgebers an dem Gelenkersatzimplantat, insbesondere am proximalen Ende eines Schaftimplantats, zur Bildung einer navigierbaren Implantat-Geber-Einheit, derart, dass aus den Gebersignalen dieser Einheit Realpositionskoordinaten des Adapters und damit wahlweise des Implantats selbst bestimmbar sind,
- eine Ausbildung der Eingabeschnittstelle zur Eingabe von Adapterparametern, die die synoptische Darstellung des Adapters bzw. des Implantats mit dem **durch** die Computertomografie-Modellbildungseinrichtung gewonnenen Abbild des Gelenk- bzw. Wirbelbereiches erlauben,
- eine Ausbildung der Matching-Verarbeitungseinheit zur Zuordnung der Realpositionskoordinaten des Adapters und wahlweise des Implantats zum Abbild des Gelenk- bzw. Wirbelbereiches im wesentlichen in Echtzeit und
- eine zur synoptischen Darstellung des Adapters bzw. Implantats in seiner Realposition mit dem an Realpositionskoordinaten angepassten Abbild des Gelenk- bzw. Wirbelbereiches ausgebildete Bilddarstellungseinheit.

11. Anordnung nach einem der vorangehenden Ansprüche,
**dadurch gekennzeichnet, dass**
der oder mindestens ein Mehrpunktgeber als passiver Vierpunktgeber mit vier Kugelreflektorabschnitten ausgebildet ist und der oder einer Stereokamera eine Beleuchtungseinrichtung zur Beleuchtung des Mehrpunktgebers oder der Mehrpunktgeber räumlich starr zugeordnet ist.

12. Anordnung nach einem der Ansprüche 2 bis 11,
**dadurch gekennzeichnet, dass**
die Benutzerschnittstelle Mittel zur Realisierung einer Menüführung mindestens zur Ausrichtung des dreidimensionalen Abbildes bezüglich der relevanten Körperachsen aufweist.

13. Anordnung nach Anspruch 12,
**dadurch gekennzeichnet, dass**
die Benutzerschnittstelle einen Mehrbereichsspeicher zur Speicherung der Implantatparameter der einsetzbaren Implantate oder eine Datenbank-Schnittstelle zu einer Implantatparameter-Datenbank aufweist und die Mittel zur Realisierung einer Menüführung zur Ausführung eines interaktiven Auswahlvorganges einer Komponente unter mehrfachem Zugriff auf den Mehrbereichsspeicher bzw. die Implantatparameter-Datenbank ausgebildet sind.

14. Anordnung nach einem der Ansprüche 3 bis 13,
**gekennzeichnet durch**
eine mit der Auswertungseinheit und der Matching-Verarbeitungseinheit verbundene Steuersignal-Erzeugungseinheit, die zu einem Vergleich eines Satzes von über die Eingabeschnittstelle eingegebenen und an die Realpositionskoordinaten des Gelenk- bzw. Wirbelbereiches angepassten Implantatpositionsdaten bzw. -ausrichtungsdaten mit aktuell erfassten Realpositionskoordinaten des Wirkabschnitts des Resektionsinstruments oder Montagewerkzeugs oder Implantats und zur Bestimmung einer Abweichung zwischen Soll- und Ist-Positionskoordinaten und zur Ausgabe von Abweichungsdaten oder eines aus der Abweichung abgeleiteten Steuerbefehls, insbesondere per Text- oder Sprachausgabe und/oder in einer synoptischen Darstellung mit dem Abbild, ausgebildet ist.

## Claims

1. Arrangement for the intra-operative determination of the spatial position and angular position of a joint replacement implant, especially a hip socket or shoulder socket or an associated stem implant, or a vertebral replacement implant, especially a lumbar or cervical vertebral implant, using a computer tomography method, having:
- a computer tomography modeling device for generating and storing a three-dimensional image of a joint region or vertebral region to be provided with the joint replacement implant,
- an optical coordinate-measuring arrangement for providing real position coordinates of defined real or virtual points of the joint region or vertebral region and/or position reference vectors between such points within the joint region or vertebral region or from those points to joint-function-relevant points on an extremity outside the joint region or vertebral region,
the coordinate-measuring arrangement comprising a stereocamera or stereocamera arrangement for the spatial recording of transducer signals, at least one multipoint transducer, which comprises a group of measurement points rigidly connected to one another, and an evaluation unit for evaluating sets of measurement point coordinates supplied by the multipoint transducer(s) and recorded by the stereocamera, and
**characterized by**
- a matching-processing unit for real position matching of the image to the actual current spatial position of the joint region or vertebral region with reference to the real position coordinates of the defined points, the matching-processing unit being configured for calculating transformation parameters with minimalization of the normal spacings.

2. Arrangement according to claim 1,
**characterized in that**
the matching-processing unit is configured for carrying out an interactive adjustment procedure for matching a sensed bone surface to a corresponding virtual surface of the image with the combined application of the principle of triangular meshing and a spatial spline approach with the definition of unknowns as spline parameters.

3. Arrangement according to claim 1 or 2,
**characterized by**
an input interface for entering implant parameters of a predetermined set of suitable implants and for specifying possible implant positions and alignments in relation to the image, which interface is connected to the computer tomography modeling device and is especially in the form of an interactive user interface having means for user guidance,
the matching-processing unit being connected to the input interface and being configured for determining desired coordinates or a desired movement vector of the implant being installed and of a resection area or resectioning instrument therefor from at least one set of entered implantation parameters, positions and alignments.

4. Arrangement according to claim 3,
**characterized in that**
the input interface is configured for the inputting and image integration of the relevant body axis vectors and the implant parameters of a hip socket, especially the coordinates of the center of rotation as well as the anteversion angle and the abduction angle.

5. Arrangement according to any one of the preceding claims,
**characterized in that**
a first multipoint transducer of the coordinate-measuring arrangement is in the form of a movable hand-guided sensor for sensing bony references in the joint region or vertebral region in order to determine the coordinates thereof.

6. Arrangement according to any one of the preceding claims,
**characterized in that**
a second multipoint transducer is configured for rigid attachment to a bone or vertebra in the joint region or vertebral region, respectively.

7. Arrangement according to any one of the preceding claims,
**characterized by**
- a resectioning instrument, especially a milling tool or a rasp, which can be rigidly connected to the second or a third multipoint transducer to form a geometrically calibrated, navigable tool/transducer unit, so that from the transducer signals of that unit there can be determined real position coordinates of an operational part of the resectioning instrument, especially a milling head or a rasp part, and therefrom, as desired, real position coordinates of a resection zone produced with the resectioning instrument,
- an input interface configured for entering instrument parameters of the resectioning instrument which allow its synoptic display with the image of the joint region or vertebral region obtained by the computer tomography modeling device,
- a matching-processing unit configured for allocating the real position coordinates of the operational part and, as desired, the real position coordinates of the resection zone to the image of the joint region or vertebral region substantially in real time, and
- an image-display unit configured for synoptic display of the operational part or resection zone in its current position with the image of the joint region or vertebral region matched to real position coordinates.

8. Arrangement according to any one of the preceding claims,
**characterized by**
- a mounting tool, especially a screwing tool, which can be rigidly connected to the second or third multipoint transducer to form a geometrically calibrated, navigable tool/transducer unit, so that the transducer signals of that unit can be used to determine real position coordinates of an operational part of the mounting tool, especially a screwdriver blade, and thus, as desired, of the implant itself,
- an input interface configured for entering tool parameters of the mounting tool which allow its synoptic display with the image of the joint region or vertebral region obtained by the computer tomography modeling device,
- a matching-processing unit configured for allocating the real position coordinates of the operational part and, as desired, of the implant to the image of the joint region or vertebral region substantially in real time, and
- an image-display unit configured for synoptic display of the operational part or implant in its real position with the image of the joint region or vertebral region matched to real position coordinates.

9. Arrangement according to claim 7 or 8,
**characterized in that**
the resectioning instrument and/or the mounting tool is in the form of a hand-guided tool with a handgrip having an attachment portion for rigid connection to the multipoint transducer.

10. Arrangement according to any one of claims 1 to 7,
**characterized by**
- an adapter component for the rigid attachment of a multipoint transducer to the joint replacement implant, especially at the proximal end of a stem implant, in order to create a navigable implant/transducer unit, so that the transducer signals of that unit can be used to determine real position coordinates of the adapter and thus, as desired, of the implant itself,
- an input interface configured for entering adapter parameters which allow synoptic display of the adapter or of the implant with the image of the joint region or vertebral region obtained by the computer tomography modeling device,
- a matching-processing unit configured for allocating the real position coordinates of the adapter and, as desired, of the implant to the image of the joint region or vertebral region substantially in real time, and
- an image-display unit configured for synoptic display of the adapter or implant in its real position with the image of the joint region or vertebral region matched to real position coordinates.

11. Arrangement according to any one of the preceding claims,
**characterized in that**
the multipoint transducer or at least one multipoint transducer is in the form of a passive four-point transducer having four spherical reflector parts and the stereocamera or a stereocamera is in spatially fixed association with an illuminating device for illuminating the multipoint transducer(s).

12. Arrangement according to any one of claims 2 to 11,
**characterized in that**
the user interface has means for providing menu guidance at least for the alignment of the three-dimensional image in relation to the relevant body axes.

13. Arrangement according to claim 12,
**characterized in that**
the user interface has a multi-region memory for storing the implant parameters of suitable implants or a data bank interface to an implant parameter data bank, and the means for providing menu guidance are configured for carrying out an interactive process of selecting a component with repeated access to the multi-region memory or to the implant parameter data bank.

14. Arrangement according to any one of claims 3 to 13,
**characterized by**
a control signal generation unit which is connected to the evaluation unit and to the matching-processing unit and which is configured for comparing a set of implant position data or alignment data that has been entered by means of the input interface and matched to the real position coordinates of the joint region or vertebral region with currently acquired real position coordinates of the operational part of the resectioning instrument or mounting tool or implant and for determining any variance between desired position and actual position coordinates and for outputting variance data or a control command derived from the variance, especially by means of a text or speech output and/or in a synoptic display with the image.

## Revendications

1. Dispositif de détermination peropératoire de la position et de l'orientation dans l'espace d'une prothèse articulaire, en particulier d'une cavité cotyloïde de la hanche ou de l'épaule ou d'une prothèse de diaphyse relative ou d'une prothèse de vertèbres, notamment d'une prothèse pour vertèbres lombaires ou cervicales, avec emploi d'un procédé de tomographie assistée par ordinateur, lequel comprend :
- un dispositif de modélisation avec tomographie assistée par ordinateur pour la production et l'enregistrement d'une image en trois dimensions d'une zone articulaire ou vertébrale à équiper de la prothèse articulaire,
- un dispositif optique de mesure des coordonnées pour la fourniture de coordonnées sur la position réelle de points définis réels ou virtuels de la zone articulaire ou vertébrale et/ou de vecteurs de relation positionnelle entre de tels points à l'intérieur de la zone articulaire ou vertébrale ou de ces points relatifs à la fonction articulaire à une extrémité, à l'extérieur de la zone articulaire ou vertébrale,
sachant que le dispositif de mesure des coordonnées comprend une caméra stéréo ou un dispositif à caméra stéréo pour le captage spatial de signaux d'émission, au moins un émetteur multipoint, qui comporte un groupe de points de mesure reliés de manière fixe, et une unité d'analyse pour l'analyse de quantités de coordonnées des points de mesure fournies par le ou les émetteurs multipoint et relevées par la caméra stéréo et **caractérisé par** :
- une unité de traitement avec matching pour l'adaptation, à la position réelle, de l'image à l'emplacement spatial actuel réel de la zone articulaire ou vertébrale au moyen des cordonnées de position réelle des points définis, sachant que l'unité de traitement avec matching est conçue pour le calcul de paramètres de transformation, avec minimisation des écarts de normales.

2. Dispositif selon la revendication 1, **caractérisé en ce que** l'unité de traitement avec matching est conçue pour l'exécution d'un procédé de compensation interactif pour l'adaptation d'une surface osseuse palpée à une surface virtuelle correspondante de l'image avec utilisation combinée du principe du maillage des triangles et d'une approche spatiale « spline » avec la détermination des inconnues en tant que paramètres spline.

3. Dispositif selon la revendication 1 ou 2,
**caractérisé par** une interface de saisie, pour la saisie de paramètres relatifs à la prothèse d'une quantité prédéterminée de prothèses pouvant être installées et pour la spécification de positions et d'orientations possibles pour la prothèse relativement à l'image, interface de saisie reliée au dispositif de modélisation avec tomographie assistée par ordinateur, réalisée en particulier sous la forme d'une interface utilisateur interactive avec des moyens de guidage de l'opérateur,
sachant que l'unité de traitement avec matching est reliée à l'interface de saisie et est conçue pour la détermination de coordonnées théoriques ou d'un vecteur de mouvement théorique de la prothèse à installer ainsi que d'une zone de résection ou d'un instrument de résection pour celui-ci à partir d'au moins un jeu de paramètres, de positions et d'orientations d'implantation saisis.

4. Dispositif selon la revendication 3,
**caractérisé en ce que** l'interface de saisie est conçue pour la saisie et l'intégration d'image des vecteurs des axes corporels pertinents et des paramètres de prothèse d'une cavité cotyloïde de la hanche, notamment des coordonnées du centre de rotation ainsi que de l'angle d'antéversion et de l'angle d'abduction.

5. Dispositif selon l'une quelconque des revendications précédentes, **caractérisé en ce qu'**un premier émetteur multipoint du dispositif de mesure des coordonnées est réalisé sous forme de palpeur mobile, guidé à la main, pour la palpation de références osseuses dans la zone articulaire ou vertébrale pour la détermination de leurs coordonnées.

6. Dispositif selon l'une quelconque des revendications précédentes, **caractérisé en ce qu'**un second émetteur multipoint est conçu pour la fixation rigide à un os ou à une vertèbre dans la zone articulaire ou vertébrale.

7. Dispositif selon l'une quelconque des revendications précédentes, **caractérisé par** :
- un instrument de résection, notamment une fraise ou une lime, qui peut être assemblé de manière fixe au second émetteur multipoint ou à un troisième pour former une unité outil-émetteur pouvant naviguer, calibrée du point de vue géométrique, de telle sorte que, à partir des signaux d'émission de cette unité, il soit possible de déterminer des coordonnées de position réelle d'un segment actif de l'instrument de résection, notamment d'une tête de fraise ou d'un segment de lime, et, à partir de cela, au choix, des coordonnées de position réelle d'un segment de résection réalisé avec l'instrument de résection,
- une configuration de l'interface de saisie pour la saisie de paramètres de l'instrument de résection qui permettent sa représentation synoptique avec l'image de la zone articulaire ou vertébrale, obtenue au moyen du dispositif de modélisation avec tomographie assistée par ordinateur,
- une configuration de l'unité de traitement avec matching pour l'attribution des coordonnées de position réelle du segment actif et, au choix, des coordonnées de la position réelle du segment de résection à l'image de la zone articulaire ou vertébrale sensiblement en temps réel et
- une unité graphique conçue pour la représentation synoptique du segment actif ou du segment de résection dans sa position actuelle avec l'image, adaptée aux coordonnées de position réelle, de la zone articulaire ou vertébrale.

8. Dispositif selon l'une quelconque des revendications précédentes, **caractérisé par** :
- un outil de montage, notamment un outil de vissage, qui peut être assemblé de manière fixe au second ou troisième émetteur multipoint pour former une unité outil-émetteur pouvant naviguer, calibrée du point de vue géométrique, de telle sorte que, à partir des signaux d'émission de cette unité, il soit possible de déterminer des coordonnées de position réelle d'un segment actif de l'outil de montage, notamment d'une lame de tournevis, et, ainsi, au choix, de la prothèse même,
- une configuration de l'interface de saisie pour la saisie de paramètres de l'outil de montage qui permettent sa représentation synoptique avec l'image de la zone articulaire ou vertébrale, obtenue au moyen du dispositif de modélisation avec tomographie assistée par ordinateur,
- une configuration de l'unité de traitement avec matching pour l'attribution des coordonnées de position réelle du segment actif et, au choix, de la prothèse à l'image de la zone articulaire ou vertébrale sensiblement en temps réel et
- une unité graphique conçue pour la représentation synoptique du segment actif ou de la prothèse dans sa position réelle avec l'image de la zone articulaire ou vertébrale, adaptée aux coordonnées de position réelle.

9. Dispositif selon la revendication 7 ou 8, **caractérisé en ce que** l'instrument de résection et/ou l'outil de montage est réalisé sous la forme d'un outil guidé à la main avec un manche, lequel comporte un segment de fixation pour l'assemblage fixe à l'émetteur multipoint.

10. Dispositif selon l'une quelconque des revendications 1 à 7, **caractérisé par** :
- une pièce d'adaptation pour la fixation rigide d'un émetteur multipoint à la prothèse articulaire, en particulier à l'extrémité proximale d'une prothèse de diaphyse, pour la formation d'une unité prothèse-émetteur pouvant naviguer de telle sorte qu'il soit possible de déterminer, à partir des signaux d'émission de cette unité, des coordonnées de position réelle de l'adaptateur et ainsi, au choix, de la prothèse même,
- une configuration de l'interface de saisie pour la saisie de paramètres de l'adaptateur qui permettent la représentation synoptique de l'adaptateur ou de la prothèse avec l'image de la zone articulaire ou vertébrale, obtenue au moyen du dispositif de modélisation avec tomographie assistée par ordinateur,
- une configuration de l'unité de traitement avec matching pour l'attribution des coordonnées de position réelle de l'adaptateur et, au choix, de la prothèse à l'image de la zone articulaire ou vertébrale sensiblement en temps réel et
- une unité graphique conçue pour la représentation synoptique de l'adaptateur ou de la prothèse dans sa position réelle avec l'image de la zone articulaire ou vertébrale, adaptée aux coordonnées de position réelle.

11. Dispositif selon l'une quelconque des revendications précédentes, **caractérisé en ce que** l'émetteur multipoint ou un émetteur multipoint minimal est réalisé sous la forme d'émetteur à quatre points passif comportant quatre segments à réflecteur sphérique et **en ce qu'**à la ou à une caméra stéréo est attribué de manière fixe, dans l'espace, un dispositif d'éclairage pour l'éclairage de l'émetteur multipoint ou des émetteurs multipoint.

12. Dispositif selon l'une quelconque des revendications 2 à 11, **caractérisé en ce que** l'interface utilisateur comporte des moyens pour la réalisation d'un guidage par menu au moins pour l'orientation de l'image en trois dimensions par rapport aux axes corporels pertinents.

13. Dispositif selon la revendication 12, **caractérisé en ce que** l'interface utilisateur comporte une mémoire multizone pour l'enregistrement des paramètres des prothèses pouvant être installées ou une interface de base de données relative à une base de données des paramètres de la prothèse et que les moyens de réalisation d'un guidage par menu sont conçus pour la réalisation d'un processus interactif de sélection d'une composante avec accès multiple à la mémoire multizone ou à la base de données des paramètres de prothèse.

14. Dispositif selon l'une quelconque des revendications 3 à 13, **caractérisé par** une unité de production de signaux de commande reliée à l'unité d'analyse et à l'unité de traitement avec matching qui est conçue pour la comparaison d'un jeu de données sur la position ou l'orientation de la prothèse, saisies par le biais de l'interface de saisie et adaptées aux coordonnées de la position réelle de la zone articulaire ou vertébrale à des coordonnées de position réelle actuellement relevées du segment actif de l'instrument de résection ou de l'outil de montage ou de la prothèse et pour la détermination d'une différence entre des coordonnées de la position théorique et réelle et pour l'émission de données de différence ou d'un ordre de commande découlant de la différence, en particulier par sortie écrite ou vocale et/ou suivant une représentation synoptique avec image.
